# EUROPEAN PATENT APPLICATION

(11) **EP 3 878 900 A1**
(43) Date of publication of application: **15.09.2021**
(21) Application number: 21161692.5
(22) Date of filing: 10.03.2021
(51) Int. Cl.: C08K 5/00, C08K 5/47, C09K 21/10, C07D 417/02, C07D 417/14

(54) **SULFENAMIDE FLAME RETARDANTS**

(30) Priority: 11.03.2020 DE 102020106689
(71) Applicant: Songwon Industrial Co., Ltd., Ulsan 680-090 (KR)
(72) Inventor: Le Gal, André, 8500 Frauenfeld (CH)
(74) Representative: Michalski Hüttermann & Partner Patentanwälte mbB

(57) **Abstract**

The invention relates to a sulfenamide flame retardant comprising a benzothiazole moiety.

## Description

Flame retardant(s) are added to both synthetic and natural polymeric materials to enhance the flame retardant properties of the polymers. Both inorganic and organic compounds have been used for achieving the flame retardant properties in various types of polymers. The main types of flame retardants include halogenated hydrocarbons, phosphorous containing compounds, metallic compounds such as metal oxides and hydroxides, and mela- mine derivatives. Halogenated flame retardants are commonly used due to their effectiveness. Nevertheless, the use of halogenated compounds has generally become of an environmental concern.

Therefore it is an object to provide new non-halogenated flame retardants with good properties.

This object is solved by a flame retardant according to Claim 1 of the present invention. Accordingly a flame retardant is provided, comprising a compound comprising at least one moiety according to structure (I) 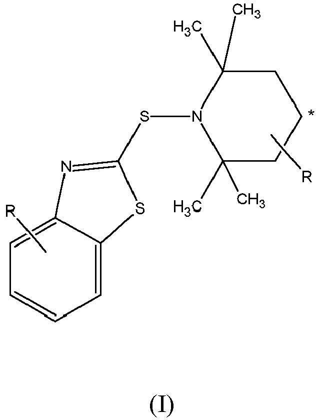 whereby "*" denotes the atom by which the moiety is linked to the further parts of the compound and "R" one or more optional alkyl substituents, whereby when more than one substituent is present, they are selected indepently from each other.

Surprisingly it has been found that by using such a flame-retardant for many application one or more of the following advantages can be observed:
- Many flame-retardants according to the present invention show a rather high melting temperature, which makes it useful for many applications, especially in construction
- Many flame retardants according to the present invention are melt processable in a temperature range corresponding to conventional processing methods of plastics materials, e.g. extrusion, injection molding, blow molding as well as fiber spinning
- Many flame retardants according to the present invention exhibit a high temperature stability which allows use in a large variety of large volume polymers, e.g. polyolefins, polyamides, polyesters, styrenics...
- Many flame-retardants according to the present invention show a rather high temperature stability, which is needed for many applicatons
- For many applications both high flammability standards with high glow wire performance at the same time can be achieved.

According to a preferred embodiment the carbon marked with "*" is linked to the further parts of the compound via a Carbon-Oxygen, Carbon-Nitrogen, or Carbon-Carbon bond, independently selected for each moiety in case more than one moiety is present.

According to a preferred embodiment, the moiety according to structure (I) is linked to the further parts of the compound via an alkyl, ether, amine or carboxy group, independently selected for each moiety in case more than one moiety is present.

According to a preferred embodiment the compound comprises more than one of the moieties of structure (I), preferably it comprises two to hundred, preferably up to twenty, more preferred up to ten and and most preferred up to eight moieties.

According to a preferred embodiment the compound comprises a backbone structure to which the moieties according to structure (I) are attached.

Preferably said backbone comprises an alkyl, polyether, polyester, polyamide, polycarbonate or polyurethane structure.

Preferably the moieties according to structure (I) are linked to said backbone via linker or spacer molecules, which preferably contain an alkyl, phenyl, ether, ester or amide group.

According to a preferred embodiment, the compound has the following structure (II) 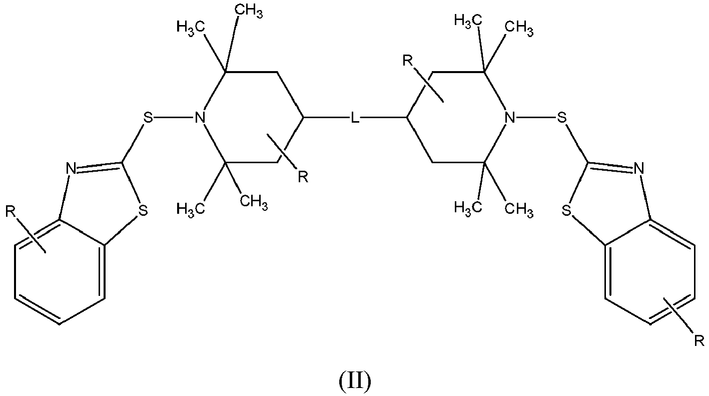 whereby R is as above and L is selected from -O-, -O-CO-O-, O-R¹-O, -O-CO-R²-O- and -O-CO-R³-CO-O-, whereby R¹ to R³ are independently from each other unsubstituted or alkyl-substituted alkyl or phenyl.

The term "alkyl" in the sense of the present invention especially means linear or branched C₁ to C₁₂ alkyl, especially linear C₁ to C₁₂ alkyl, furthermore especially means linear or branched C₂ to C₈ alkyl, and more preferred linear C₂ to C₈ alkyl and most preferred linear C₃ to C₇ alkyl.

The term "alkyl- substituted" in the sense of the present invention especially means methyl or ethyl-substituted, preferably methyl-substituted. The term "alkyl substituents" in the sense of the present invention is to be understood analagous.

According to a preferred embodiment L is -O-CO-O- or -O-CO-R³-CO-O-. According to a preferred embodiment, L is -O-CO-(CₘH₂ₘ)-CO-O with m being from 4 to 12, more preferred 5, 6 or 7.

The invention furthermore relates to a composition, which comprises (a) a compound of the formula (I) as defined herein and (b) a polymer substrate.

Preferably the weight ratio of compound (a) to (b) (wt/wt) is ≥ 0.1% to ≤ 5%, preferably ≥ 0,5% to ≤ 1.5%.

A suitable polymer substrate (b) may comprise natural and/or synthetic polymers, such as:
1. Polymers of mono- and diolefins, for example polypropylene, polyisobutylene, polybutene-1 , poly-4-methylpentene-1 , polyvinylcyclohexane, polyisoprene or polybutadiene and also polymerisates of cycloolefins, for example of cyclopentene or norbornene; and also polyethylene (which may optionally be crosslinked), for example high density poly- ethylene (HDPE), high density polyethylene of high molecular weight (HDPE-HMW), high density polyethylene of ultra-high molecular weight (HDPE-UHMW), medium density polyethylene (MDPE), low density polyethylene (LDPE), and linear low density polyethylene (LLDPE), (VLDPE) and (ULDPE).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of mono- and di-olefins with one another or with other vinyl monomers, for example ethylene/propylene copolymers, linear low density polyethylene (LLDPE) and mixtures thereof with low density polyethylene (LDPE), propylene/butene-1 copolymers, propylene/isobutylene copolymers, ethylene/butene-1 copolymers, ethylene/hexene copolymers, ethylene/- methylpentene copolymers, ethylene/heptene copolymers, ethylene/octene copolymers, ethylene/vinylcyclohexane copolymers, ethylene/cycloolefin copolymers, for example ethylene/norbornene (COC), ethylene/1 -olefin copolymers wherein the 1 -olefin is prepared in situ, propylene/butadiene copolymers, isobutylene/isoprene copolymers, ethylene/vinyl cyclohexene copolymers, eth- ylene/alkyl acrylate copolymers, ethylene/alkyl methacrylate copolymers, ethylene/vinyl acetate copolymers, ethylene/acrylic acid copolymers and salts thereof (ionomers), and also terpolymers of ethylene with propylene and a diene, such as hexadiene, dicyclopentadiene or ethylidenenorbornene; and also mixtures of such copolymers with one another or with polymers mentioned under 1), for example polypropylene-ethylene/propylene copolymers, LDPE- ethylene/vinyl acetate copolymers, LDPE-ethylene/acrylic acid copolymers, LLDPE-ethylene/vinyl acetate copolymers, LLDPE-ethylene/acrylic acid copol- ymers and alternately or randomly structured polyalkylene-carbon monoxide copolymers and mixtures thereof with other polymers, for example polyamides.
4. Polystyrene, poly(p-methylstyrene), poly(a-methylstyrene).
5. Aromatic homopolymers and copolymers derived from vinyl- aromatic monomers, for example styrene, a-methylstyrene, all isomers of vinyl- toluene, for example p-vinyltoluene, all isomers of ethylstyrene, propylstyrene, vinylbiphenyl, vinylnaphthalene, vinylan- thracene and mixtures thereof; homo- polymers and copolymers can have a syndiotactic, isotactic, hemi-isotactic or atactic stereo structure; preference is given to atactic polymers. Also included are stereo block polymers.
6. Homopolymers and copolymers can have a syndiotactic, isotactic, hemi-isotactic or atactic stereo structure; preference is given to atactic polymers. Also included are stereo block polymers.
   a) Copolymers including the already mentioned vinyl-aromatic monomers and co- monomers selected from ethylene, propylene, dienes, nitriles, acids, maleic anhydrides, maleic acid amides, vinyl acetate, vinyl chloride and acrylic acid derivatives and mixtures thereof, for example styrene/butadiene, styrene/acrylonitrile, styrene/ethylene (interpolymers), styrene/alkyl methacry- late, styrene/butadiene/alkyl acrylate and methacrylate, styrene/maleic anhydride, styrene/acrylonitrile/methyl acrylate; high-impact-strength mixtures consisting of styrene copolymers and another polymer, for example a polyacrylate, a diene polymer or an ethylene/propylene/dieneterpolymer; and also block copolymers of styrene, for example styrene/butadiene/styrene, styrene/iso- prene/styrene, styrene/ethylenebutylene/styrene or styrene/ethylene-propyl- ene/styrene.
   b) Hydrogenated aromatic polymers prepared by hydrogenation of the polymers mentioned under item 6), especially polycyclohexylethylene (PCHE), often also referred to as polyvinylcyclohexane (PVCH), which is prepared by hydrogenation of atactic polystyrene.
   c) Hydrogenated aromatic polymers prepared by hydrogenation of the polymers mentioned under item 6a).
7. Graft copolymers of vinyl-aromatic monomers, for example styrene on polybutadiene, styrene on polybutadiene/styrene or polybutadiene/- acrylonitrile copolymers, styrene and acrylonitrile (or methacrylonitrile) on polybutadiene; styrene, acrylonitrile and me- thyl methacrylate on polybutadiene; styrene and maleic anhydride on polybutadiene; styrene, acrylonitrile and maleic anhydride or maleic acid imide on polybutadiene; styrene and maleic acid imide on polybutadiene, styrene and alkyl acrylates or alkyl meth- acry- lates on polybutadiene, styrene and acrylonitrile on ethylene/propylene/diene ter- polymers, styrene and acrylonitrile on polyalkyl acrylates or polyalkyl meth- acrylates, styrene and acrylonitrile on acrylate/butadiene copolymers, and mixtures thereof with the copolymers mentioned above under Paragraph 6, such as those known, for example, as so-called ABS, MBS, ASA or AES polymers.
8. Halogen-containing polymers, for example polychloroprene, chlorinated rubber, chlorinated and brominated copolymer of isobutylene/isoprene (halobutyl rubber), chlorinated or chlorosulphonated polyethylene, copolymers of ethylene and chlorinated ethylene, epichlorohydrin homo- and co-polymers, especially polymers of halogen-containing vinyl compounds, for example polyvinyl chloride, polyvinylidene chloride, polyvinyl fluoride, polyvinylidene fluoride; and copolymers thereof, such as vinyl chloride/vinylidene chloride, vinyl chloride/vinyl acetate or vinylidene chloride/vinyl acetate.
9. Polymers derived from α,β-unsaturated acids and derivatives thereof, such as polyacrylates and polymethacrylates, or polymethyl methacryl- ates, polyacrylamides and polyacrylon itriles impact-resistant-modified with butyl acrylate.
10. Copolymers of the monomers mentioned under Paragraph 9 with one another or with other unsaturated monomers, for example acrylonitrile/butadiene copolymers, acrylonitrile/alkyl acrylate copolymers, acrylonitrile/alkoxyalkyl/acrylate copolymers, acrylonitrile/vinyl halide copolymers or acrylonitrile/alkyl methacrylate/butadiene terpolymers.
11. Polymers derived from unsaturated alcohols and amines or their acyl derivatives or acetals, such as polyvinyl alcohol, polyvinyl acetate, stearate, benzoate or maleate, polyvinylbutyral, polyallyl phthalate, polyallylmelamine; and the copolymers thereof with olefins mentioned under item 1 .
12. Homo- and co-polymers of cyclic ethers, such as polyalkylene glycols, polyethylene oxide, polypropylene oxide or copolymers thereof with bisglycidyl ethers.
13. Polyacetals, such as polyoxymethylene, and also those polyox-ymethylenes which contain comonomers, for example ethylene oxide; polyacetals modified with thermoplastic polyurethanes, acrylates or MBS.
14. Polyphenylene oxides and sulphides and mixtures thereof with styrene polymers or polyamides. 15. Polyamides and copolyamides derived from diamines and dicar- boxylic acids and/or from aminocarboxylic acids or the corresponding lactams, such as polyamide 4, poly- amide 6, polyamide 6/6, 6/10, 6/9, 6/12, 4/6, 12/12, polyamide 1 1 , polyamide 12, aromatic polyamides derived from m-xylene, diamine and adipic acid; polyamide 6/1 (polyhexamethylene isophthalimide, MXD (m-xylylenediamine); polyamides prepared from hexamethylenediamine and iso- and/or terephthalic acid and optionally an elastomer as modifier, for example poly-2,4,4-trimethylhexamethylene terephthalamide or poly-m-phenylene isophthalamide. Block copolymers of the above-mentioned polyamides with polyolefins, olefin copolymers, ionomers or chemically bonded or grafted elastomers; or with polyethers, for example with polyethylene glycol, polypropylene glycol or polytetra- methylene glycol. Also polyamides or copolyamides modified with EPDM or ABS; and polyamides condensed during processing ("RIM polyamide systems").

Examples of polyamides and copolyamides that can be used are derived from, inter alia, ε-caprolactam, adipic acid, sebacic acid, dodecanoic acid, isophthalic acid, terephthalic acid, hexamethylenediamine, tetrameth- ylenediamine, 2-methyl-pentamethylenediamine, 2,2,4-trimethylhexamethylenediamine, 2,4,4-trimethylhexamethyl enediamine, m-xylylenediamine or bis- (3-methyl-4-aminocyclohexyl)methane; and also semi-aromatic polyamides such as polyamide 66/61 , for example consisting of 70 - 95% polyamide 6/6 and 5 - 30% polyamide 6/1; and also tricopolymers in which some of the polyamide 6/6 has been replaced, for example consisting of 60 - 89% polyamide 6/6, 5 - 30% polyamide 6/I and 1 - 10% of another aliphatic polyamide; the latter may consist of, for example, polyamide 6, polyamide 1 1 , polyamide 12 or polyamide 6/12 units. Such tricopolymers may accordingly be designated polyamide 66/6I/6, polyamide 66/61/11, polyamide 66/61/12, polyamide 66/61/610 or polyamide 66/61/612.
16. Polyureas, polyimides, polyamide imides, polyether imides, polyester imides, polyhydantoins and polybenzimidazoles.
17. Polyesters derived from dicarboxylic acids and dialcohols and/or from hydroxycarboxylic acids or the corresponding lactones, such as polyethylene terephthalate, polypropylene terephthalate, polybutylene terephthalate, poly-1 ,4-dimethylolcyclohexane terephthalate, polyalkylene naphthalate (PAN) and polyhydroxy-benzoates, and also block polyether esters derived from pol- yethers with hydroxyl terminal groups; and also polyesters modified with polycarbonates or MBS.
18. Polycarbonates and polyester carbonates.
19. Mixtures (polyblends) of the afore-mentioned polymers, for example PP/EPDM, polyamide/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPO/HIPS, PPO/PA 6.6 and copolymers, PA/HDPE, PA/PP, PA/PPO, PBT/PC/ABS or PBT/PET/PC. Preference is given to compositions wherein the thermoplastic polymer is high-impact polystyrene (HIPS), expandable polystyrene (EPS), extruded polystyrene (XPS), polyphenylene ether (PPE), polyamide, polyester, polycarbonate (PC) or a polymer blend of the type ABS (acrylo- nitrile-butadiene-styrene) or PC/ABS (polycarbonate/acrylonitrile-butadiene-styrene) or PPE/HIPS (polyphenylene ether/high-impact polystyrene), especally a polyamide, polyester or a PPE/HIPS blend. Special preference is given to polymer compositions according to the invention that comprise a filler or a reinforcing agent, especially glass-fibre-reinforced polymers, e.g. glass-fibre- reinforced polyamide.
20. Natural polymers, for example cellulose, starch (amylose and amylopectin), lignocellulose, proteins silk, polyhydroxyalkanoates, polypeptides, polysacharides: Xanthan gum, B-Glucans, chitosan and natural rubbers.
21 . Bio polymers, for example polycaprolactones, polylactides, poly(Lactide-co-Glycolide) Copolymers (PLGA), poly(glycolic acid) (PGA) and polydioxanone (PDS).

A preferred embodiment relates to flame retardant compositions, wherein the polymer substrate (b) consists of polystyrene, polystyrene copolymers, polyethylene, polypropylene or blends of polypropylene with polyolefins. Examples are blends of polypropylene with polyethylene selected from the group consisting of high density polyethylene (HDPE), high molecular weight high density polyethylene (HMW HDPE), ultra high molecular weight high density polyethylene (UHMW HDPE), medium density polyethylene (MDPE), low density polyethylene (LDPE), linear low density polyethylene (LLDPE), branched low density polyethylene (BLDPE) and ethylene-propylene-diene ter- polymers (EPDM) containing small proportions of diene.

The present invention further relates to a flame retardant composition, which comprises, in addition to the components (a) and (b), as defined above, (c) further additives selected from the group consisting of polymer stabilizers and additional non-halogenated flame-retardants, such as melamine containing flame retardants, phosphorus containing flame-retardants, further nitrogen containing flame-retardants other than melamine containing flame retardants, and inorganic flame-retardants.

Stabilizers are preferably halogen-free and selected from nitroxyl stabilizers, nitrone stabilizers, amine oxide stabilizers, benzofuranone stabilizers, phosphite and phosphonite stabilizers, quinone methide stabilizers and monoacrylate esters of 2,2'-alkylidenebisphenol stabilizers.

Additional flame retardants as of present component (c) are known components, items of commerce or can be obtained by known methods.

Representative melamine containing flame retardants are for example, melamine comprising compounds, wherein the melamine structure: 1 ,3,5- triazine-2,4,6-triamin (= cyanuric acid triamide) or condensates thereof are present. The definition applies to monomeric, oligomeric or polymeric compounds of melamine, condensates of melamine or condensates with of melamine and phosphoric thereof.

Preferred melamine comprising compounds are melamine cyanu- rate, melamine phosphate, dimelamine phosphate, melamine pyrophosphate, melamine polyphosphate, melamine phenyl phosphonate, melamine borate, melamine ammonium phosphate, melamine ammonium polyphosphate, melamine ammonium pyrophosphate, melem, melam or melon or polyphosphates of melem, melam or melon.

Representative phosphorus containing flame-retardants are for example: Organic metal phosphinates (Aluminium phosphinates, Exolit OP, Clar- iant), pentaerythritol phosphates, tetraphenyl resorcinol diphosphite (FYROLFLEX<®>RDP, Akzo Nobel), tetrakis(hydroxymethyl)phosphonium sulphide, triphenyl phosphate, diethyl-N,N-bis(2-hydroxyethyl)-aminomethyl phosphonate, hydroxyalkyl esters of phosphorus acids and cyclic phospho- nates (ADK STAB FP 600/ 800/ 2200, Adeka Corp), AFLAMMIT PCO 900 /800 /700, (Thor GmbH), ammonium polyphosphate (APP). (EXOLIT AP 766, Clari- ant) or (HOSTAFLAM<®>AP750, Clariant), resorcinol diphosphate oligomer (RDP), phosphazene flame-retardants, 9,10-dihydro-9-oxa-10-phosphaphen- anthrene-10-oxide) (DOPO) or its derivatives, di(ethylenediamine) phosphate (DEDAP), and ethylenediamine diphosphate (EDAP) or their mixtures (e.g. BUDIT 3167, Budenheim). Further nitrogen containing flame retardants other than melamine containing flame retardants are, for example, isocyanurate flame-retardants, such as polyisocyanurate, esters of isocyanuric acid or isocyanurates, melamine metal phosphates (SAFIRE® 200 /400 /600, Floridienne Chimie). Repre- sentative examples are hydroxyalkyl isocyanurates, such as tris-(2-hydroxy- ethyl)isocyanurate, tris(hydroxymethyl)isocyanurate, tris(3-hydroxy-n-proyl)iso- cyanurate or triglycidyl isocyanurate. Further examples are: benzoguanamine, tris(hydroxyethyl) isocyanurate, allantoin, glycouril, melamine cyanurate, urea cyanurate, poly- [2,4 -(piperazine- I ,4-yl)-6-(morpholine-4-yl)-1 ,3,5-triazine]/pip- erazin (MCA® PPM TRIAZINE HF, MCA Technologies) azoalkanes and related compounds (e.g.AZONOR, azine, azoxy, hydrazone, triazenyl, INAZO), NOR compounds (FLAMESTAB® NOR 1 16, TINUVIN™ NOR 371 , BASF), or ammonium polyphosphate. The sulfenamides can also be used together with synergists based on other radical generators e.g. disulfides or peroxides.

Representative organohalogen flame-retardants are, for example:
Polybrominated diphenyl oxide (DE-60F, Great Lakes Corp.), decabromodi- phenyl ethane (SAYTEX™ 8010, Albemarle), hexabromocyclododecane (SAYTEX™ HP 900P , Albemarle), brominated polymers and oligomers such as styrene-butadiene block copolymers (EMERALD INNOVATION™ 3000, Chemtura, GREEN ARMOR™, GREEN CREST™, Albemarle, FR-122P™, ICL), polyphenylene oxide and its derivatives, brominated polyacrylates (FR- 1025 P™,ICL), decabromodiphenyl oxide (DBDPO; SAYTEX<®>102E), tris[3-bromo-2,2-bis(bromomethyl)propyl] phosphate (PB 370<®>, FMC Corp.), tris(2,3-dibromopropyl)phosphate, tris(2,3-dichloropropyl)phosphate, chlorendic acid, tetrachlorophthalic acid, tetrabromophthalic acid, 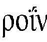 -β-chloroethyl triphos- phonate mixture, tetrabromobisphenol A bis(2,3-dibromopropyl ether) (PE68), brominated epoxy resin, ethylene-bis(tetrabromophthalimide) (SAYTEX<®>BT- 93), bis(hexachlorocyclopentadieno)-cyclooctane (DECHLORANE PLUS<®>), chlorinated paraffins, octabromodiphenyl ether, hexa-chlorocyclopentadiene derivatives, 1 ,2-bis(tribromophenoxy)ethane (FF680), tetrabromo-bisphenol A (SAYTEX<®>RB100), ethylene bis-(dibromo-norbornanedicarboximide) (SAYTEX<®>BN-451), bis-(hexachlorocycloentadeno) cyclooctane, PTFE, tris-(2,3- dibromopropyl)-iso-cyanurate, and ethylene-bis-tetrabromophthalimide.

The flame-retardant mentioned above routinely combined with inorganic (hydr)oxide synergists. Most common for this use are aluminum (hydr)- oxide, such as AI(OH)3or AIOOH, magnesium hydroxide, zinc or antimony oxides, e.g. Sb2O3or Sb2O5. Boron compounds are suitable, too.

The above-mentioned additional flame retardant compounds are advantageously contained in the composition of the invention in an amount (weight) from about 0.25% to about 45.0% by weight of the organic polymer substrate; for instance about 0.25% to about 35.0%; for example about 0.25% to about 30.0% by weight of the polymer.

As mentioned above, the composition according to the invention may additionally contain one or more conventional additives, for example se- lected from pigments, dyes, plasticizers, anti-dripping agents such as fluorinat- ed polymers (PTFE, Metablen A3800, Mitsubishi Rayon), nanoclays (Cloisite 30B) and borates (FIREBRAKE®, Borax), antioxidants, thixotropic agents, levelling assistants, basic co-stabilizers, metal passivators, metal oxides, organo-phosphorus compounds, further light stabilizers and mixtures thereof, especial- ly pigments, phenolic antioxidants, calcium stearate, zinc stearate, UV absorbers of the 2-hydroxybenzophenone, 2-(2'-hydroxyphenyl)benzotriazole and/or 2-(2-hydroxyphenyl)-1,3,5-triazine groups.

The additives mentioned above are preferably contained in an amount (weight) of 0.01 to 10.0%, especially 0.05 to 5.0%, relative to the weight of the polymer substrate b).

According to a preferred embodiment of the invention, the composition may additionally comprise one or more fillers. Example of fillers according to the invention are glass beads, hollow glass spheres, amorphous silica, chalk, mica, calcinated kaolin, wollastonite, talc, magnesium carbonate, barium sulphate or similar products and they may have been surface treated with fatty acids or the like or may have been milled in presence of fatty acids or the like. Any particulate substance currently available in the market as a filler for thermoplastic resins may as well be used in the compositions according to the present invention, provided that the average particle size of the powder, measured by laser instrument, is in the range of about 2 microns to 20 microns.

The amont of fillers mentioned above are preferably contained in an amount (weight) of 1 to 40.0%, especially 5 to 25.0%, relative to the weight of the polymer substrate b).

The present invention accordingly relates also to the use of the compounds of formula (I) as defined above for imparting flame-resistant properties to a polymer substrate, for example synthetic polymers, especially to thermoplastics.

The present invention also relates to a method of imparting flame-resistant properties to synthetic polymers, wherein at least one compound of formula (I) according to the invention is incorporated in the polymer substrate or is applied to their surface.

The incorporation of the compounds of formula (I) and the optional additional components, as defined above, into the polymer substrate is carried out by known methods such as dry blending in the form of a powder, or wet mixing in the form of solutions, dispersions or suspensions for example in an inert solvent, water or oil. The compound of formula (I) and optional further additives may be incorporated, for example, before or after molding or also by applying the dissolved or dispersed additive or additive mixture to the polymer material, with or without subsequent evaporation of the solvent or the suspension/dispersion agent. They may be added directly into the processing appa- ratus (e.g. extruders, internal mixers, etc.), e.g. as a dry mixture or powder, or as a solution or dispersion or suspension or melt.

The present invention furthermore relates to a product comprising an inventive flame retardant and/or a product making use of the inventive use.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the subclaims and the following description of the respective examples. These examples do not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

### EXAMPLES

In the following the invention shall be described according to two inventive examples which are for illustrative purposes only and non-binding.
Inventive Example I: The compound according to structure (II) with L = O-CO-O and R= absent
Inventive Example II: The compound according to structure (II) with L = O-CO- (CH₂)₆-COO- and R= absent.

For both examples, the melting point (via DSC) and the thermal stability (1% loss in TGA under N₂) was measured. The results are shown in Table I

**Table I**

| | Example I | Example II |
|---|---|---|
| Melting point (°C, via DSC) | 262 | 165 |
| Thermal stability (°C, 1% loss in TGA under N2) | 284 | 285 |

As can be seen from the data, both compounds show excellent properties, making them especially useful as flame-retardants for a broad range of applications.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A flame retardant, comprising a compound comprising at least one moiety according to structure (I) 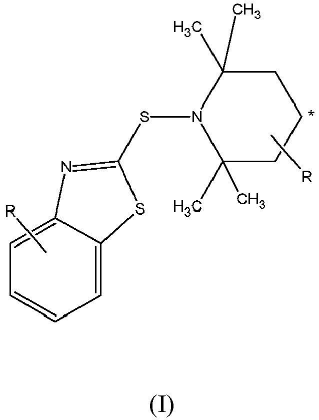 whereby "*" denotes the atom by which the moiety is linked to the further parts of the compound and "R" one or more optionally alkyl substituents, whereby when more than one substituent is present, they are selected indepently from each other.

2. The flame retardant according to Claim 1, whereby the carbon marked with "*" is linked to the further parts of the compound via a Carbon-Oxygen, Carbon-Nitrogen, or Carbon-Carbon bond, independently selected for each moiety in case more than one moiety is present.

3. The flame retardant according to claim 1 or 2, whereby the moiety according to structure (I) is linked to the further parts of the compound by an alkyl, ether, amine or carboxy group, independently selected for each moiety in case more than one moiety is present

4. The flame retardant according to any of the claims 1 to 3, whereby the compound comprises more than one of the moieties of structure (I)

5. The flame retardant according to any of the claims 1 to 4, comprising a compound of structure (II) 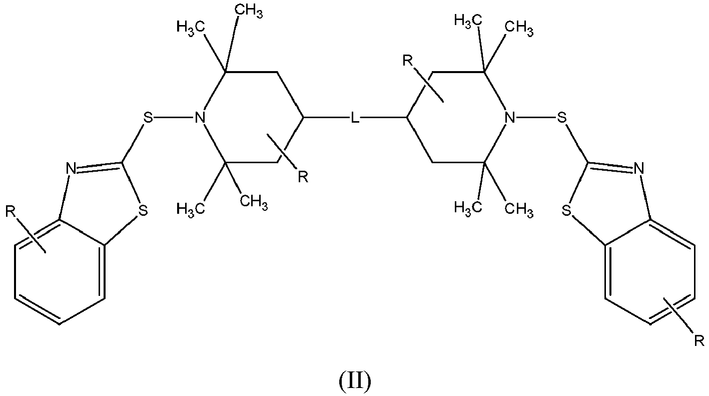 whereby R is as above and L is selected from -O-, -O-CO-O-, O-R¹-O, -O-CO-R²-O- and -O-CO-R³-CO-O-, whereby R¹ to R³ are independently from each other unsubstituted or alkyl-substituted alkyl or phenyl.

6. The flame retardant according to Claim 5, whereby L is selected from L is -O-CO-O- or -O-CO-R³-CO-O-.

7. The flame retardant according to Claim 5, whereby L is -O-CO-(CₘH₂ₘ)-CO-O with m being 5, 6 or 7.

8. A composition, which comprises (a) a compound of the formula (I) according to any of the claims 1 to 7 and (b) a polymer substrate

9. Use of a compounds of formula (I) according to any of the claims 1 to 7 for imparting flame-resistant properties to a polymer substrate.

10. Product comprising a compound of formula (I) according to any of the claims 1 to 7, a composition of claim 8 or making use of claim 9.
